# EUROPÄISCHE PATENTSCHRIFT

(11) **EP 1 235 549 B1**
(45) Veröffentlichungstag und Bekanntmachung des Hinweises auf die Patenterteilung: **29.11.2006**
(21) Anmeldenummer: 00971379.3
(22) Anmeldetag: 14.10.2000
(51) Int. Cl.: A61Q 5/10, A61K 8/34, A61K 8/49

(54) **MITTEL ZUM FÄRBEN VON KERATINHALTIGEN FASERN**
AGENT FOR COLOURING KERATIN CONTAINING FIBRES
AGENT POUR COLORER DES FIBRES A BASE DE KERATINE

(30) Priorität: 23.10.1999 DE 19951134
(43) Veröffentlichungstag der Anmeldung: 04.09.2002
(73) Patentinhaber: Henkel Kommanditgesellschaft auf Aktien, 40589 Düsseldorf (DE)
(72) Erfinder: MÖLLER, Hinrich, 40789 Monheim (DE); OBERKOBUSCH, Doris, 40591 Düsseldorf (DE); HÖFFKES, Horst, 40595 Düsseldorf (DE)
(86) Internationale Anmeldenummer: PCT/EP2000/010125
(87) Internationale Veröffentlichungsnummer: WO 2001/034106

(56) Entgegenhaltungen:
- EP-A- 0 820 759
- EP-A- 0 873 743
- EP-A- 0 873 745
- WO-A-00/15183
- WO-A-00/38634
- WO-A-99/59528
- DE-A- 19 820 894
- US-A- 4 391 603
- US-A- 5 199 954

## Beschreibung

Die Erfindung betrifft ein Mittel zum Färben von keratinhaltigen Fasern, insbesondere menschlichen Haaren, das eine Kombination aus aromatischen Aldehyden und CH-aktiven Verbindungen enthält, die Verwendung dieser Kombination als färbende Komponente in Haarfärbemitteln sowie ein Verfahren zum Färben von keratinhaltigen Fasern, insbesondere menschlichen Haaren.

Für das Färben von keratinhaltigen Fasern, z. B. Haaren, Wolle oder Pelzen, kommen im allgemeinen entweder direktziehende Farbstoffe oder Oxidationsfarbstoffe, die durch oxidative Kupplung einer oder mehrerer Entwicklerkomponenten untereinander oder mit einer oder mehreren Kupplerkomponenten entstehen, zur Anwendung. Kuppler- und Entwicklerkompönenten werden auch als Oxidationsfarbstoffvorprodukte bezeichnet.

Als Entwicklerkomponenten werden üblicherweise primäre aromatische Amine mit einer weiteren, in para- oder ortho-Position befindlichen freien oder substituierten Hydroxy- oder Aminogruppe, Diaminopyridinderivate, heterocyclische Hydrazone, 4-Aminopyrazolonderivate sowie 2,4,5,6-Tetraaminopyrimidin und dessen Derivate eingesetzt.

Spezielle Vertreter sind beispielsweise p-Phenylendiamin, p-Toluylendiamin, 2,4,5,6-Tetraäminopyrimidin, p-Aminophenol, N,N-Bis-(2-hydroxyethyl)-p-phenylendiamin, 2-(2,5-Diaminophenyl)-ethanol, 2-(2,5-Diaminophenoxy)-ethanol, 1-Phenyl-3-carboxyamido-4-amino-pyrazolon-5, 4-Amino-3-methylphenol, 2-Aminomethyl-4-aminophenol, 2-Hydroxymethyl-4-aminophenol, 2-Hydroxy-4,5,6-triaminopyrimidin, 2,4-Dihydroxy-5,6-diaminopyrimidin und 2,5,6-Triamino-4-hydroxypyrimidin.

Als Kupplerkomponenten werden in der Regel m-Phenylendiaminderivate, Naphthole, Resorcin und Resorcinderivate, Pyrazolone, m-Aminophenote und substituierte Pyridinderivate verwendet. Als Kupplersubstanzen eignen sich insbesondere α-Naphthol, 1,5-, 2,7- und 1,7-Dihydroxynaphthalin, 5-Amino-2-methylphenol, m-Aminophenol, Resorcin, Resorcinmonomethylether, m-Phenylendiamin, 2,4-Diaminophenoxyethanol, 2-Amino-4-(2-hydroxyethylamino)-anisol (Lehmanns Blau), 1-Phenyl-3-methyl-pyrazolon-5, 2,4-Dichlor-3-aminophenol, 1,3-Bis-(2,4-diaminophenoxy)-propan, 2-Chlorresorcin, 4-Chlorresorcin, 2-Chlor-6-methyl-3-aminophenol, 2-Methylresorcin, 5-Methylresorcin, 3-Amino-6-methoxy-2-methylamino-pyridin und 3,5-Diamino-2,6-dimethoxypyridin.

Bezüglich weiterer üblicher Farbstoffkomponenten wird ausdrücklich auf die Reihe "Dermatology", herausgeben von Ch. Culnan, H. Maibach, Verlag Marcel Dekker Inc., New York, Basel, 1986, Bd. 7, Ch. Zviak, The Science of Hair Care, Kap. 7, Seiten 248 - 250 (Direktziehende Farbstoffe), und Kap. 8, Seiten 264 - 267 (Oxidationsfarbstoffe), sowie das "Europäische Inventar der Kosmetikrohstoffe", 1996, herausgegeben von der Europäischen Kommission, erhältlich in Diskettenform vom Bundesverband der deutschen Industrie- und Handelsunternehmen für Arzneimittel, Reformwaren und Körperpflegemittel e.V., Mannheim, Bezug genommen.

Mit Oxidationsfarbstoffen lassen sich zwar intensive Färbungen mit guten Echtheitseigenschaften erzielen, die Entwicklung der Farbe geschieht jedoch i.a. unter dem Einfluß von Oxidationsmitteln wie z. B. H₂O₂, was in einigen Fällen Schädigungen der Faser zur Folge haben kann. Desweiteren können einige Oxidationsfarbstoffvorprodukte bzw. bestimmte Mischungen von Oxidationsfarbstoffvorprodukten bisweilen bei Personen mit empfindlicher Haut sensibilisierend wirken. Direktziehende Farbstoffe werden unter schonenderen Bedingungen appliziert, ihr Nachteil liegt jedoch darin, daß die Färbungen häufig nur über unzureichende Echtheitseigenschaften verfügen.

Die Verwendung der unten näher beschriebenen Kombination aus aromatischen Aldehyden und CH-aktiven Verbindungen zum Färben von keratinhaltigen Fasern ist bislang nicht bekannt.

Die Druckschrift EP-A2-873 743 betrifft Mittel zur Färbung keratinhaltiger Fasern, die mindestens ein Benzylidenketon enthalten, das gegebenenfalls zur Erweiterung der Farbpalette mit mindestens einer Verbindung mit einer primären oder sekundären Amino- oder Hydroxygruppe und/oder mindestens einer CH-aktiven Verbindung kombiniert werden kann.

Aufgabe der vorliegenden Erfindung ist es, Färbemittel für Keratinfasern, insbesondere menschliche Haare, bereitzustellen, die hinsichtlich der Farbtiefe, der Grauabdeckung und den Echtheitseigenschaften qualitativ den üblichen Oxidationshaarfärbemitteln mindestens gleichwertig sind, ohne jedoch unbedingt auf Oxidationsmittel wie z. B. H₂O₂ angewiesen zu sein. Darüber hinaus dürfen die Färbemittel kein oder lediglich ein sehr geringes Sensibilisierungspotential aufweisen.

Überraschenderweise wurde nun gefunden, daß die Kombination aus den in der Formel I dargestellten aromatischen Aldehyden und CH-aktiven Verbindungen der Formel II sich auch in Abwesenheit von oxidierenden Agentien hervorragend zum Färben von keratinhaltigen Fasern eignen. Sie ergeben Ausfärbungen mit hervorragender Brillanz und Farbtiefe und führen zu vielfältigen Farbnuancen. Der Einsatz von oxidierenden Agenzien soll dabei jedoch nicht prinzipiell ausgeschlossen werden.

Gegenstand der Erfindung ist ein Mittel zum Färben von keratinhaltigen Fasern, insbesondere menschlichen Haaren, enthaltend eine Kombination aus aromatischen Aldehyden mit der Formel I, 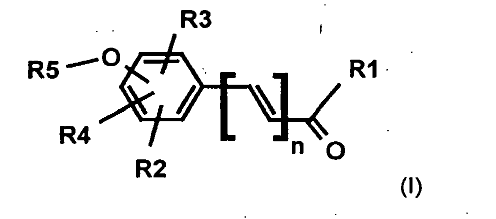 in der R¹ ein Wasserstoffatom ist,
R², R³ und R⁴ unabhängig voneinander ein Wasserstoffatom, eine C₁₋₄-Alkylgruppe, ein Halogenatom, eine Hydroxy-, C₁₋₄-Alkoxy- oder Nitrogruppe bedeuten und zwei dieser Gruppen auch für einen ankondensierten aromatischen Ring stehen können,
R⁵ ein Wasserstoffatom, eine C₁₋₄-Alkyl-, C₂₋₄-Hydroxyalkyl-, C₁₋₄-Alkenyl- oder Arylgruppe bedeutet oder gemeinsam mit R², R³ oder R⁴ einen ankondensierten fünf- bis siebengliedrigen heterocylischen Ring bildet, oder gemeinsam mit dem Sauerstoffatom eine Olatgruppe bildet, deren negative Ladung durch ein Alkali- oder Ammoniumion ausgeglichen ist, und
n für die Zahlen 0, 1 oder 2 steht,
und CH-aktiven Verbindungen mit der Formel II, 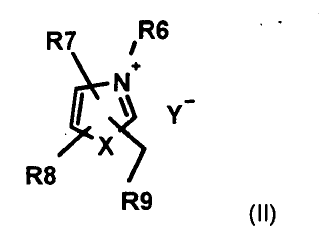 in der R⁶ eine C₁₋₁₀-Alkyl-, C₂₋₄-Alkenyl-, C₂₋₄-Hydroxyalkyl-, C₂₋₄-Carboxyalkyl-, C₂₋₄-Sulfoalkyl- oder Aralkylgruppe ist,
R⁷ und R⁸ unabhängig voneinander ein Wasserstoffatom, eine C₁₋₄-Alkylgruppe, ein Halogenatom, eine Hydroxy-, C₁₋₄-Alkoxy- oder Nitrogruppe bedeuten oder zusammen einen ankondensierten aromatischen Ring bilden,
R⁹ ein Wasserstoffatom, eine C₁₋₄-Alkyl- oder eine Arylgruppe,
X ein Sauerstoff- oder Schwefelatom, die Gruppe -CH=CH- oder >N-R¹², in der
R¹² für eine C₁₋₄-Alkyl-, C₂₋₄-Carboxyalkyl-, C₂₋₄-Sulfoalkyl-, C₂₋₄-Sulfoxyalkyl-, C₂₋₄-Hydroxyalkyl- oder Aralkylgruppe steht, ist und
Y⁻ für ein Anion steht, das ausgewählt ist aus Halogenid, C₁₋₄-Alkylsulfat. C₁₋₄-Alkansulfonat, Arensulfonat, C₁₋₄-Perfluoralkansulfat, Tetrafluorborat, Perhalogenat, Sulfat, Hydrogensulfat oder Carboxylat,
und gegebenenfalls zusätzlich eine CH-aktive Verbindung des Formel (III) 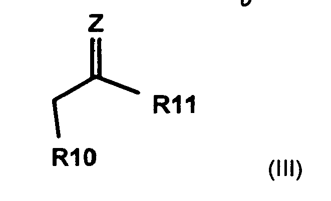 in der R¹⁰ für eine C₁₋₄-Acylgruppe, Aroyl-, C₁₋₄-Alkylsulfonyl-, C₁₋₄-Alkylsulfinyl-, C₁₋₄-Alkylamino-, Di-C₁₋₄-Alkylamino-, Vinylcarbonyl-, Methinimino-, Nitril-, Ester- oder Carbonsäureamidgruppe, die ggf. durch C₁₋₄-Alkyl-, C₂₋₄-Hydroxyalkyl- oder Arylgruppen substituiert sein kann, steht und
R¹¹ für eine C₁₋₄-Acyl-, C₁₋₄-Alkoxy-, C₁₋₄-Alkylamino-, C₁₋₄-Acylamino- oder Di-C₁₋₄-alkylaminogruppe steht,
wobei die Reste R¹⁰ und R¹¹ gemeinsam mit dem Restmolekül einen 5-, 6- oder 7-gliedrigen Heterocyclus aus der Reihe der Thiazolidin-2,5-dione, Thiazolidin-2-thion-5-one, Perhydropyrimidin-2,4,6-trione, Perhydropyrimidin-2-thion-4,6-dione, 2-Pyrazolin-5-one, 1,2-Dihydro-6-hydroxy-2-hydroxypyridine, Benzothiazin-3-one oder deren Enolester bilden können, und
Z für Sauerstoff, Schwefel oder die Dicyanmethylengruppe steht.

Unter keratinhaltigen Fasern sind Wolle, Pelze, Federn und insbesondere menschliche Haare zu verstehen. Die erfindungsgemäßen Färbemittel können prinzipiell aber auch zum Färben anderer Naturfasern, wie z. B. Baumwolle, Jute, Sisal, Leinen oder Seide, modifizierter Naturfasern, wie z. B. Regeneratcellulose, Nitro-, Alkyl- oder Hydroxyalkyl-oder Acetylcellulose und synthetischer Fasern, wie z. B. Polyamid-, Polyacrylnitril-, Polyurethan- und Polyesterfasern verwendet werden.

Bevorzugt eingesetzte Verbindungen mit der Formel I sind z. B. Salicylaldehyd, 3-Hydroxybenzaldehyd, 4-Hydroxybenzaldehyd, o-Anisaldehyd, m-Anisaldehyd, p-Anisaldehyd, 4-Hydroxy-3-methylbenzaldehyd, 2-Hydroxy-3-methylbenzaldehyd, 2-Hydroxy-5-methylbenzaldehyd, 2-Hydroxy-4-methylbenzaldehyd, 2,3-Dihydroxybenzaldehyd, 2,5-Dihydroxybenzaldehyd, 3,4-Dihydroxybenzaldehyd, 2,4-Dihydroxybenzaldehyd, 3,5-Dihydroxybenzaldehyd, 2,3-Dihydrobenzo[b]furan-5-carboxaldehyd, Piperonal, 4-Ethoxy-benzaldehyd, 3,5-Dimethyl-4-hydroxybenzaldehyd; Vanillin, Isovanillin, 2,3,4-Trihydroxybenzaldehyd, 2,4,5-Trihydroxybenzaldehyd, 2,4,6-Trihydroxybenzaldehyd, 3,4,5-Trihydroxybenzaldehyd, 3-Chlor-4-hydroxybenzaldehyd, 2,4-Dimethoxybenzaldehyd, 2,5-Dimethoxybenzaldehyd, 2,6-Dimethoxybenzaldehyd, 3,4-Dimethoxybenzaldehyd, 3,4-Dihydroxy-5-methoxybenzaldehyd, 2-Hydroxy-1-naphthaldehyd, 4-Hydroxy-1-naphthaldehyd, 1- Hydroxy-2-naphthaldehyd, 2-Methoxy-1-naphthaldehyd, 4-Methoxy-1-naphthaldehyd, 2-Hydroxyzimtaldehyd, 4-Hydroxyzimtaldehyd, 2,4-Dihydroxyzimtaldehyd, 4-Hydroxy-3-methoxyzimtaldehyd (Coniferylaldehyd), 3,5-Dimethoxy-4-hydroxyzimtaldehyd sowie deren beliebigen Gemische.

Als Beispiele für bevorzugt eingesetzte Verbindungen mit der Formel II können genannt werden: 1,4-Dimethylchinolinium-, 1,2-Dimethylchinolinium-, 1,4-Dimethylpyridinium-, 1,2-Dimethylpyridinium-, 2-Methyl-1-ethylchinolinium-, 2,3-Di-methylisochinolinium-, 1,2,3,3-Tetramethyl-3H-indolium-, 2,3-Dimethylbenzothiazolium-, 2,3-Dimethyl-6-nitrobenzothiazolium-, 3-Benzyl-2-benzothiazolium-, 2-Methyl-3-propylbenzothiazolium-, 2,4-Dimethyl-3-ethylthiazolium-, 3-(2-Carboxyethyl)-2,5-dimethylbenzothiazolium-, 1,2,3-Trimethylbenzimidazolium-, 5,6-Dichlor-1,3-diethyl-2-methylbenzimidazolium-, 3-Ethyl-2-methylbenzothiazolium-, 3-Ethyl-2-methylnaphtho[ 1,2-d]thiazolium-, 5-Chlor-3-ethyl-2-methylbenzothiazolium-, 3-Ethyl-2-methylbenzoxazolium-Salze, die z. B. als Chloride, Bromide, lodide, Methansulfonate, Benzolsulfonate, p-Toluolsulfonate, Trifluormethansulfonate, Methylsulfate, Tetrafluorborate vorliegen können, sowie 2-Methyl-3-(3-sulfopropyl)-benzothiazolium-hydroxid, inneres Salz, 4-Methyl-1-(3-sulfopropyl)-pyridinium-hydroxid, inneres Salz, 4-Methyl-1-(3-sulfopropyl)-chinolinium-hydroxid, inneres Salz, 5-Methoxy-2-methyl-3-(3-sulfopropyl)-benzothiazolium-hydroxid, inneres Salz und beliebige Gemische der voranstehenden.

Beispiele für Verbindungen mit der Formel III sind Rhodanin, Rhodanin-3-essigsäure, Barbitursäure, Thiobarbitursäure, 1,3-Dimethyl-, 1,3-Diethylthiobarbitursäure, Oxindol, 3-lndoxylacetat, Cumaranon, 1-Methyl-3-phenylpyrazolinon, Indan-1,3-dion, Cyclopentan-1,3-dion, 1,2-Dihydro-1-ethyl-6-hydroxy-4-methyl-2-oxo-3-pyridincarbonitril, 1-Dicyanmethylenindan, 1-Dicyanmethylenindan-3-on und beliebige Gemische der voranstehenden.

Diese Substanzen sind zum großen Teil literaturbekannt oder im Handel erhältlich oder nach bekannten Syntheseverfahren herstellbar.

Die voranstehend genannten Verbindungen mit der Formel I, Formel II bzw. Formel III werden vorzugsweise in den erfindungsgemäßen Mitteln jeweils in einer Menge von 0,03 bis 65 mmol, insbesondere von 1 bis 40 mmol, bezogen auf 100 g des gesamten Färbemittels, verwendet. Weiterhin kann es erfindungsgemäß besonders bevorzugt sein, die Verbindung der Formel I einerseits und die CH-aktiven Verbindungen der Formel II und oder III andererseits im molaren Mengenverhältnis von 2 : 1 bis 1 : 2, insbesondere etwa äquimolar, einzusetzen.

Sie können als direktziehende Färbemittel oder in Gegenwart von üblichen Oxidationsfarbstoffvorprodukten eingesetzt werden.

Färbemittel, die als färbende Komponente die erfindungsgemäße Kombination allein enthalten, werden bevorzugt für Färbungen im Gelb-, Orange-, Rot- und Violettbereich eingesetzt.

Zur Erlangung weiterer und intensiverer Ausfärbungen können die erfindungsgemäßen Mittel können zusätzlich Farbverstärker enthalten. Die Farbverstärker sind vorzugsweise ausgewählt aus der Gruppe bestehend aus Piperidin, Piperidin-2-carbonsäure, Piperidin-3-carbonsäure, Piperidin-4-carbonsäure, Pyridin, 2-Hydroxypyridin, 3-Hydroxypyridin, 4-Hydroxypyridin, Imidazol, 1-Methylimidazol, Histidin, Pyrrolidin, Prolin, Pyrrolidon, Pyrrolidon-5-carbonsäure, Pyrazol, 1,2,4-Triazol, Piperazidin, deren Derivate sowie deren physiologisch verträglichen Salzen.

Die voranstehend genannten Farbverstärker können in einer Menge von jeweils 0,03 bis 65, insbesondere 1 bis 40 mmol, jeweils bezogen auf 100 g des gesamten Färbemittels, eingesetzt werden.

In allen Färbemitteln können auch mehrere verschiedene Kombinationen aus den Verbindungen mit den Formeln I, II und gegebenenfalls III gemeinsam zum Einsatz kommen; ebenso können auch mehrere verschiedene Farbverstärker gemeinsam verwendet werden.

Auf die Anwesenheit von Oxidationsmitteln, z. B. H₂O₂, kann dabei verzichtet werden. Es kann jedoch u. U.. wünschenswert sein, den erfindungsgemäßen Mitteln zur Erzielung der Nuancen, die heller als die zu färbende keratinhaltige Faser sind, Wasserstoffperoxid oder andere Oxidationsmittel zuzusetzen. Oxidationsmittel werden in der Regel in einer Menge von 0,01 bis 6 Gew.%, bezogen auf die Anwendungslösung, eingesetzt. Ein für menschliches Haar bevorzugtes Oxidationsmittel ist H₂O₂.

In einer bevorzugten Ausführungsform enthalten die erfindungsgemäßen Färbemittel zur weiteren Modifizierung der Farbnuancen neben den erfindungsgemäß enthaltenen Verbindungen zusätzlich übliche direktziehende Farbstoffe, z. B. aus der Gruppe der Nitrophenylendiamine, Nitroaminophenole, Anthrachinone oder Indophenole, wie z. B. die unter den internationalen Bezeichnungen bzw. Handelsnamen HC Yellow 2, HC Yellow 4, HC Yellow 6, Basic Yellow 57, Disperse Orange 3, HC Red 3, HC Red BN, Basic Red 76, HC Blue 2, Disperse Blue 3, Basic Blue 99, HC Violet 1, Disperse Violet 1, Disperse Violet 4, Disperse Black 9, Basic Brown 16 und Basic Brown 17 bekannten Verbindungen sowie Pikraminsäure 2-Amino-6-chloro-4-nitrophenol, 4-Amino-2-nitrodiphenylamin-2'-carbonsäure, 6-Nitro-1,2,3,4-tetrahydrochinoxatin, 4-N-Ethyl-1,4-bis(2'-hydroxyethylamino)-2-nitrobenzol-hydrochlorid und 1-Methyl-3-nitro-4-(2'-hydroxyethyl)-aminobenzol. Die erfindungsgemäßen Mittel gemäß dieser Ausführungsform enthalten die direktziehenden Farbstoffe bevorzugt in einer Menge von 0,01 bis 20 Gew.-%, bezogen auf das gesamte Färbemittel.

Weiterhin können die erfindungsgemäßen Zubereitungen auch in der Natur vorkommende Farbstoffe wie beispielsweise Henna rot, Henna neutral, Henna schwarz, Kamillenblüte, Sandelholz, schwarzen Tee, Faulbaumrinde, Salbei, Blauholz, Krappwurzel, Catechu, Sedre und Alkannawurzel enthalten.

Es ist nicht erforderlich, daß die erfindungsgemäß enthaltenen Verbindungen der Formeln I, II und III oder die fakultativ enthaltenen Farbverstärker unddirektziehenden Farbstoffe jeweils einheitliche Verbindungen darstellen. Vielmehr können in den erfindungsgemäßen Färbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z. B. toxikologischen, ausgeschlossen werden müssen.

Die erfindungsgemäßen Färbemittel ergeben bereits bei physiologisch verträglichen Temperaturen von unter 45°C intensive Färbungen. Sie eignen sich deshalb besonders zum Färben von menschlichen Haaren. Zur Anwendung auf dem menschlichen Haar können die Färbemittel üblicherweise in einen wasserhaltigen kosmetischen Träger eingearbeitet werden. Geeignete wasserhaltige kosmetische Träger sind z. B. Cremes, Emulsionen, Gele oder auch tensidhaltige schäumende Lösungen wie z. B. Shampoos oder andere Zubereitungen, die für die Anwendung auf den keratinhaltigen Fasern geeignet sind. Falls erforderlich ist es auch möglich, die Färbemittel inwasserfreie Träger einzuarbeiten.

Weiterhin können die erfindungsgemäßen Färbemittel alle in solchen Zubereitungen bekannten Wirk-, Zusatz- und Hilfsstoffe enthalten. In vielen Fällen enthalten die Färbemittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form derNatrium-, Kalium- und Ammonium- sowie der Mono-, Di- und Trialkanolammoniumsalze mit 2 oder 3 C-Atomen in der Alkanolgruppe,
- lineare Fettsäuren mit 10 bis 22 C-Atomen (Seifen),
- Ethercarbonsäuren der Formel R-O-(CH₂-CH₂O)ₓ -CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobemsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(CH₂-CH₂O)ₓ-SO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Citronensäure mit Alkoholen, dieAnlagerungsprodukte von etwa 2 bis 15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C₈-C₂₂-Carbonsäuren, wie Ölsäure, Stearinsäure, Isostearinsäure und Palmitinsäure.

Als zwitterionische Tenside werden solche oberflächenaktiven Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniurnglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der CTFA-Bezeichnung Cocamidopropyl Betaine bekannte Fettsäureamid-Derivat.

Unter ampholytischen Tensiden werden solche oberflächenaktiven Verbindungen verstanden, die außer einer C₈₋₁₈-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Nichtionische Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂₋₂₂-Fettsäuremono- und -diester von Anlagerungsprodukten von 1 bis 30 Mol Ethylenoxid an Glycerin,
- C₈₋₂₂-Alkylmono- und -oligoglycoside und deren ethoxylierte Analoga,
- Anlagerungsprodukte von 5 bis 60 Mol Ethylenoxid an Rizinusöl und gehärtetes Rizinusöl,
- Anlagerungeprodukte von Ethylenoxid an Sorbitanfettsäureester
- Anlagerungsprodukte von Ethylenoxid an Fettsäurealkanolamide.

Beispiele für die in den erfindungsgemäßen Haarbehandlungsmitteln verwendbaren kationischen Tenside sind insbesondere quartäre Ammoniumverbindungen. Bevorzugt sind Ammoniumhalogenide wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weitere erfindungsgemäß verwendbare kationische Tenside stellen die quatemisierten Proteinhydrolysate dar.

Erfindungsgemäß ebenfalls geeignet sind kationische Silikonöle wie beispielsweise die im Handel erhältlichen Produkte Q2-7224 (Hersteller: Dow Corning; ein stabilisiertes Trimethylsilylamodimethicon), Dow Coming 929 Emulsion (enthaltend ein hydroxylamino-modifiziertes Silicon, das auch als Amodimethicone bezeichnet wird), SM-2059 (Hersteller: General Electric), SLM-55067 (Hersteller: Wacker) sowie Abil^{®}-Quat 3270 und 3272 (Hersteller: Th. Goldschmidt; diquatemäre Polydimethylsiloxane, Quaternium-80).

Alkylamidoamine, insbesondere Fettsäureamidoamine wie das unter der Bezeichnung Tego Amid^{®}S 18 erhältliche Stearylamidopropyldimethylamin, zeichnen sich neben einer guten konditionierenden Wirkung speziell durch ihre gute biologische Abbaubarkeit aus.

Ebenfalls sehr gut biologisch abbaubar sind quatemäre Esterverbindungen, sogenannte "Esterquats", wie die unter dem Warenzeichen Stepantex^{®} vertriebenen Methylhydroxyalkyldialkoyloxyalkylammoniummethosulfate.

Ein Beispiel für ein als kationisches Tensid einsetzbares quatemäres Zuckerderivat stellt das Handelsprodukt Glucquat^{®}100 dar, gemäß CTFA-Nomenklatur ein "Lauryl Methyl Gluceth-10 Hydroxypropyl Dimonium Chloride".

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Weitere Wirk-, Hilfs- und Zusatzstoffe sind beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- kationische Polymere wie quatemisierte Celluloseether, Polysiloxane mit quatemären Gruppen, Dimethyldiallylammoniumchlorid-Polymere, Acrylamid-Dimethyldiallylammoniumchlorid-Copoiymere, mit Diethylsulfat quatemierte Dimethylaminoethylmethacrylat-Vinylpyrrolidon-Copolymere, Vnylpyrrolidon-Imidazoliniummethochlorid-Copolymere und quatemierter Polyvinylalkohol,
- zwitterionische und amphotere Polymere wie beispielsweise Acrylamidopropyl-trimethytammoniumchiorid/Acryfat-Copotymere und Octylacrylamid/Methylmethacrylat/tert.-Butylaminoethylmethacrylat/2-Hydroxypropylmethacrylat-Copolymere,
- anionische Polymere wie beispielsweise Polyacrylsäuren, vemetzte Polyacrylsäuren, Vinylacetat/Crotonsäure-Copoiymere, Vinylpyrrolidon/Vinylacrylat-Copolymere, Vinylacetat/Butylmaleat/lsobomylacrylat-Copolymere, Methylvinylether/Maleinsäureanhydrid-Copolymere und Acrytsäure/Ethylacrylat/N-tert.-Butylacrylamid-Terpolymere,
- Verdickungsmittel wie Agar-Agar, Guar-Gum, Alginate, Xanthan-Gum, Gummi arabicum, Karaya-Gummi, Johannisbrotkemmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z. B. Polyvinylalkohol,
- Strukturanten wie Glucose und Maleinsäure,
- haarkonditionierende Verbindungen wie Phospholipide, beispielsweise Sojalecithin, Ei-Lecitin und Kephaline, sowie Silikonöle,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quatemisierte Proteinhydrolysate,
- Parfümöle, Dimethylisosorbid und Cyclodextrine,
- Lösungsvermittler wie Ethanol, Isopropanol, Ethylenglykol, Propylenglykol, Glycerin und Diethylenglykol,
- Antischuppenwirkstoffe wie Piroctone Olamine und Zink Omadine,
- weitere Substanzen zur Einstellung des pH-Wertes,
- Wirkstoffe wie Panthenol, Pantothensäure, Allantoin, Pyrrolidoncarbonsäuren und deren Salze, Pflanzenextrakte und Vitamine,
- Cholesterin,
- Lichtschutzmittel,
- Konsistenzgeber wie Zuckerester, Polyolester oder Polyolalkylether,
- Fette und Wachse wie Walrat, Bienenwachs, Montanwachs, Paraffine, Fettalkohole und Fettsäureester,
- Fettsäurealkanolamide,
- Komplexbildner wie EDTA, NTA und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate, Imidazole, Tannine, Pyrrol,
- Trübungsmittel wie Latex,
- Perlglanzmittel wie Ethylenglykolmono- und -distearat,
- Treibmittel wie Propan-Butan-Gemische, N₂O, Dimethylether, CO₂ und Luft sowie
- Antioxidantien.

Die Bestandteile des wasserhaltigen Trägers werden zur Herstellung der erfindungsgemäßen Färbemittel in für diesen Zweck üblichen Mengen eingesetzt; z. B. werden Emulgiermittel in Konzentrationen von 0,5 bis 30 Gew.-% und Verdickungsmittel in Konzentrationen von 0,1 bis 25 Gew.-% des gesamten Färbemittels eingesetzt.

Für das Färbeergebnis kann es vorteilhaft sein, den Färbemitteln Ammonium- oder Metallsalze zuzugeben. Geeignete Metallsalze sind z. B. Formiate, Carbonate, Halogenide, Sulfate, Butyrate, Valeriate, Capronate, Acetate, Lactate, Glykolate, Tartrate, Citrate, Gluconate, Propionate, Phosphate und Phosphonate von Alkalimetallen, wie Kalium, Natrium oder Lithium, Erdalkalimetallen, wie Magnesium, Calcium, Strontium oder Barium, oder von Aluminium, Mangan, Eisen, Kobalt, Kupfer oder Zink, wobei Natriumacetat, Lithiumbromid, Calciumbromid, Calciumgluconat, Zinkchlorid, Zinksulfat, Magnesiumchlorid, Magnesiumsulfat, Ammoniumcarbonat, -chlorid und -acetat bevorzugt sind. Diese Salze sind vorzugsweise in einer Menge von 0,03 bis 65, insbesondere von 1 bis 40, mmol bezogen auf 100 g des gesamten Färbemittels, enthalten.

Der pH-Wert der gebrauchsfertigen Färbezubereitungen liegt üblicherweise zwischen 2 und 12, vorzugsweise zwischen 4 und 10.

Ein weiterer Gegenstand der vorliegenden Erfindung betrifft die Verwendung von einer Kombination aus aromatischen Aldehyden mit der Formel I, 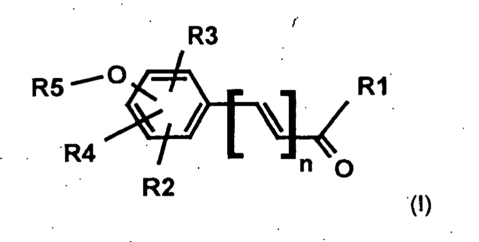 in der R¹, R², R³, R⁴, R⁵, Q und n wie oben definiert sind,
und CH-aktiven Verbindungen mit der Formel II und gegebenenfalls zusätzlich des Formel (III) 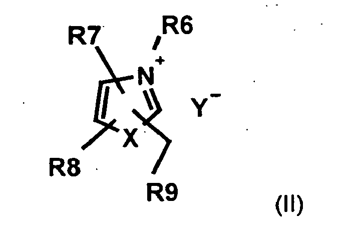 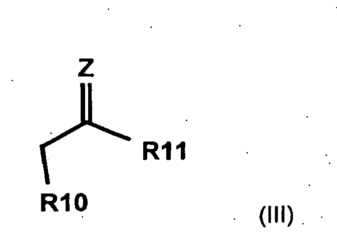 in der R⁶, R⁷, R⁸, R⁹, X, Y, R¹⁰, R¹¹ und Z wie oben definiert sind, als eine färbende Komponente in Oxidationshaarfärbemitteln.

Noch ein weiterer Gegenstand der vorliegenden Erfindung betrifft ein Verfahren zum Färben von keratinhaltigen Fasern, insbesondere menschlichen Haaren, worin ein Färbemittel, enthaltend eine Kombination aus aromatischen Aldehyden mit der Formel 1, 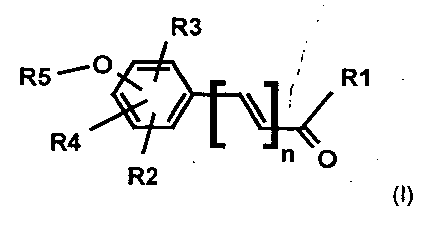 in der R¹, R², R³, R⁴, R⁵, Q und n wie oben definiert sind,
und CH-aktiven Verbindungen mit der Formel II und gegebenenfalls zusätzlich der Formel (III) 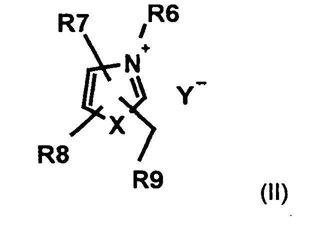 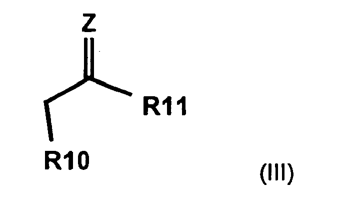 in der R⁶, R⁷, R⁸, R⁹, X, Y, R¹⁰, R¹¹ und Z wie oben definiert sind,
sowie übliche kosmetische Inhaltsstoffe, auf die keratinhaltigen Fasern aufgebracht, einige Zeit, üblicherweise ca. 30 Minuten, auf der Faser belassen und anschließend wieder ausgespült oder mit einem Shampoo ausgewaschen wird.

Die aromatischen Aldehyde der Formel I und dieCH-aktiven Verbindungen mit den Formeln II und gegebenenfalls III können entweder gleichzeitig auf das Haar aufgebracht werden oder aber auch nacheinander, wobei es unerheblich ist, welche der beiden Komponenten zuerst aufgetragen wird. Die fakultativ enthaltenen Ammonium- oder Metallsalze können dabei der ersten oder der zweiten Komponente zugesetzt werden. Zwischen dem Auftragen der ersten und der zweiten Komponente können bis zu 30 Minuten Zeitabstand liegen. Auch eine Vorbehandlung der Fasern mit der Salzlösung ist möglich.

Die aromatischen Aldehyde der Formel I und die CH-aktiven Verbindungen mit den Formeln II und gegebenenfalls III können entweder getrennt oder zusammen gelagert werden, entweder in einer flüssigen bis pastösen Zubereitung (wäßrig oderwasserfrei) oder als trockenes Pulver. Bei der getrennten Lagerung werden die Komponenten erst unmittelbar vor der Anwendung miteinander innig vermischt. Bei der trockenen Lagerung wird vor der Anwendung üblicherweise eine definierte Menge warmen (30 bis 80°C) Wassers hinzugefügt und eine homogene Mischung hergestellt.

### Beispiele

### Herstellung einer Färbelösung

Es wurde je eine Aufschlämmung von 5 mmol eines aromatischen Aldehyds mit der Formel I, 5 mmol einerCH-aktiven Verbindung mit der Formel II in 25 ml Wasser bei 50°C hergestellt. Die Aufschlämmungen wurden nach Abkühlen auf 30°C miteinander vermischt, mit 5 mmol Natriumacetat, ggf. 5 mMol Piperidin und einem Tropfen einer 20-%igen Fettalkylethersulfat-Lösung versetzt und mit verdünnter NaOH oder Salzsäure der pH-Wert entsprechend eingestellt. Die Ausfärbungen wurden bei pH 6,00 durchgeführt, es sei denn es ist etwas anderes vermerkt.

In diese Färbemischung wurde bei 30°C 30 Minuten lang eine Strähne zu 90% ergrauten, nicht vorbehandelten Menschenhaares eingebracht. Die Strähne wurde dann 30 Sek. mit lauwarmem Wasser gespült, mit warmer Luft getrocknet und anschließend ausgekämmt.

Die jeweiligen Farbnuancen und Farbtiefen sind in den nachfolgenden Tabellen wiedergegeben.

Die Farbtiefe wurde dabei nach folgender Skala bewertet:

| | | |
|---|---|---|
| - | : | keine oder eine sehr blasse Ausfärbung |
| (+) | : | schwache Intensität |
| + | : | mittlere Intensität |
| +(+) | : | mittlere bis starke Intensität |
| ++ | : | starke Intensität |
| ++(+) | : | starke bis sehr starke Intensität |
| +++ | : | sehr starke Intensität |

**Tabelle 1**

| **Aldehyd der Formel I** | **Verbindung mit der Formel II** | **Farbe** | **Intensität** |
|---|---|---|---|
| 4-Hydroxy-3-methoxyzimtaldehyd | 1-Ethyl-2-methylnaphtho[1,2-d]-thiazolium-p-toluolsulfonat | braunrot | ++(+) |
| 4-Hydroxy-3-methoxyzimtaldehyd¹ | 2H-1,4-Benzothiazin-3(4H)-on | orange | ++(+) |
| 4-Hydroxy-3-methoxyzimtaldehyd | 3-Ethyl-2-methylbenzoxazoliumiochid | orange (leuchtend) | ++ |
| 4-Hydroxy-3-methoxyzimtaldehyd¹ | 3-Ethyl-2-methylbenzoxazoliumiochid | braun-orange | ++ |
| 4-Hydroxybenzaldehyd | 1-Ethyl-2-methylchinolinium-iodid | goldgelb | ++ |
| 4-Hydroxybenzaldehyd | 1-Ethyl-2-methylchinolinium-iodid | goldgelb | ++ |
| 4-Hydroxybenzaldehyd | 3-Ethyl-2-methylbenzolthiazolium-iodid | leuchtend orange-rot | ++(+) |
| 4-Hydroxynaphthaldehyd | 1-Ethyl-4-methylchinolinium-iodid | braun-gelb | + |
| 4-Hydroxynaphthaldehyd | 3-Ethyl-2-methylbenzothiazolium-iodid | rotviolett | +++ |
| 4-Hydroxynaphthaldehyd | 1-Ethyl-2-methylnaphtho[1,2-d]-thiazolium-p-toluolsulfonat | graugrün | +(+) |
| 4-Hydroxy-3-methoxyzimtaldehyd | 1-Ethyl-2-methylchinolinium-iodid | orange | ++ |
| 4-Hydroxy-3-methoxyzimtaldehyd | 1-Ethyl-4-methyl-chinolinium-iodid | orange-braun | ++ |
| 4-Hydroxy-3-methoxyzimtaldehyd | 3-Ethyl-2-methylbenzothiazolium-iodid | violettrot | +++ |
| Vanillin | 1-Ethyl-2-methylchinolinium-iodid | braun-gelb | +(+) |
| Vanillin | 1-Ethyl-4-methylchinolinium-iodid | orange-gelb | + |
| Vanillin | 3-Ethyl-2-methylbenzothiazolium-iodid | neutral-rot | ++(+) |
| Vanillin | 1,2 Diemethylnaphtho[1,2-d]-thiazolium-p-toluolsulfonat | braun-orange | ++ |

| | | | |
|---|---|---|---|
| 1 Ausfärbung bei pH 9,00 und unter Zusatz einer äquimolaren Menge von Piperidin als Farbverstärker | | | |

## Patentansprüche

1. Mittel zum Färben von keratinhaltigen Fasern, insbesondere menschlichen Haaren, enthaltend als färbende Komponente eine Kombination aus aromatischen Aldehyden mit der Formel I, 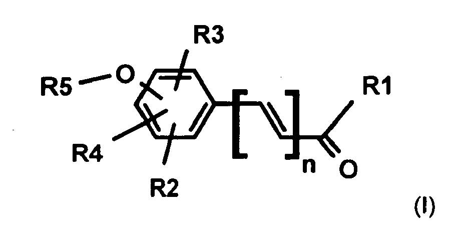 in der R¹ ein Wasserstoffatom ist,
R², R³ und R⁴ unabhängig voneinander ein Wasserstoffatom, eine C₁₋₄-Alkylgruppe, ein Halogenatom, eine Hydroxy-, C₁₋₄-Alkoxy- oder Nitrogruppe bedeuten und zwei dieser Gruppen auch für einen ankondensierten aromatischen Ring stehen können,
R⁵ ein Wasserstoffatom, eine C₁₋₄-Alkyl-, C₂₋₄-Hydroxyalkyl-, C₁₋₄-Alkenyl- oder Arylgruppe bedeutet oder gemeinsam mit R², R³ oder R⁴ einen ankondensierten fünf- bis siebengliedrigen heterocylischen Ring bildet, oder gemeinsam mit dem Sauerstoffatom eine Olatgruppe bildet, deren negative Ladung durch ein Alkali- oder Ammoniumion ausgeglichen ist, und
n für die Zahlen 0, 1 oder 2 steht, und CH-aktiven Verbindungen mit der Formel II, 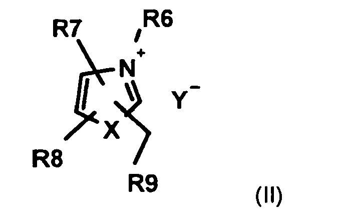 in der R⁶ eine C₁₋₁₀-Alkyl-, C₂₋₄-Alkenyl-, C₂₋₄-Hydroxyalkyl-, C₂₋₄-Carboxyalkyl-, C₂₋₄-Sulfoalkyl- oder Aralkylgruppe ist,
R⁷ und R⁸ unabhängig voneinander ein Wasserstoffatom, eine C₁₋₄-Alkylgruppe, ein Halogenatom, eine Hydroxy-, C₁₋₄-Alkoxy- oder Nitrogruppe bedeuten oder zusammen einen ankondensierten aromatischen Ring bilden,
R⁹ ein Wasserstoffatom, eine C₁₋₄-Alkyl- oder eine Arylgruppe,
X ein Sauerstoff- oder Schwefelatom, die Gruppe -CH=CH- oder >N-R¹², in der R¹² für eine C₁₋₄-Alkyl-, C₂₋₄-Carboxyalkyl-, C₂₋₄-Sulfoalkyl-, C₂₋₄-Sulfoxyalkyl-, C₂₋₄-Hydroxyalkyl- oder Aralkylgruppe steht, ist und
Y⁻ für ein Anion steht, das ausgewählt ist aus Halogenid, C₁₋₄-Alkylsulfat, C₁₋₄-Alkansulfonat, Arensulfonat, C₁₋₄-Perfluoralkansulfat, Tetrafluorborat, Perhalogenat, Sulfat, Hydrogensulfat oder Carboxylat.

2. Mittel nach Anspruch 1, **dadurch gekennzeichnet, daß** es zusätzlich CH-aktive Verbindungen gemäß Formel III enthält, 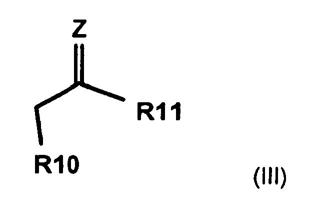 in der R¹⁰ für eine C₁₋₄-Acylgruppe, Aroyl-, C₁₋₄-Alkylsulfonyl-, C₁₋₄-Alkylsulfinyl-, C₁₋₄-Alkylamino-, Di-C₁₋₄-Alkylamino-, Vinylcarbonyl-, Methinimino-, Nitril-, Ester- oder Carbonsäureamidgruppe, die ggf. durch C₁₋₄-Alkyl-, C₂₋₄-Hydroxyalkyl- oder Arylgruppen substituiert sein kann, steht und
R¹¹ für eine C₁₋₄-Acyl-, C₁₋₄-Alkoxy-, C₁₋₄-Alkylamino-, C₁₋₄-Acylamino- oder Di-C₁₋₄-alkylaminogruppe steht,
wobei die Reste R¹⁰ und R¹¹ gemeinsam mit dem Restmolekül einen 5-, 6- oder 7-gliedrigen Heterocyclus aus der Reihe der Thiazolidin-2,5-dione, Thiazolidin-2-thion-5-one, Perhydropyrimidin-2,4,6-trione, Perhydropyrimidin-2-thion-4,6-dione, 2-Pyrazolin-5-one, 1,2-Dihydro-6-hydroxy-2-hydroxypyridine, Benzothiazin-3-one oder deren Enolester bilden können,
Z für Sauerstoff, Schwefel oder die Dicyanmethylengruppe steht.

3. Mittel nach Anspruch 1 oder 2, **dadurch gekennzeichnet, daß** als Verbindungen mit der Formel I Salicylaldehyd, 3-Hydroxybenzaldehyd, 4-Hydroxybenzaldehyd, o-Anisaldehyd, m-Anisaldehyd, p-Anisaldehyd, 4-Hydroxy-3-methylbenzaldehyd, 2-Hydroxy-3-methylbenzaldehyd, 2-Hydroxy-5-methylbenzaldehyd, 2-Hydroxy-4-methylbenzaldehyd, 2,3-Dihydroxybenzaldehyd, 2,5-Dihydroxybenzaldehyd, 3,4-Dihydroxybenzaldehyd, 2,4-Dihydroxybenzaldehyd, 3,5-Dihydroxybenzaldehyd, 2,3-Dihydrobenzo[b]furan-5-carboxaldehyd, Piperonal, 4-Ethoxy-benzaldehyd, 3,5-Dimethyl-4-hydroxybenzaldehyd, Vanillin, Isovanillin, 2,3,4-Trihydroxybenzaldehyd, 2,4,5-Trihydroxybenzaldehyd, 2,4,6-Trihydroxybenzaldehyd, 3,4,5-Trihydroxybenzaldehyd, 3-Chlor-4-hydroxybenzaldehyd, 2,4-Dimethoxybenzaldehyd, 2,5-Dimethoxybenzaldehyd, 2,6-Dimethoxybenzaldehyd, 3,4-Dimethoxybenzaldehyd, 3,4-Dihydroxy-5-methoxybenzaldehyd, 2-Hydroxy-1-naphthaldehyd, 4-Hydroxy-1-naphthaldehyd, 1-Hydroxy-2-naphthaldehyd, 2-Methoxy-1-naphthaldehyd, 4-Methoxy-1-naphthaldehyd, 2-Hydroxyzimtaldehyd, 4-Hydroxyzimtaldehyd, 2,4-Dihydroxyzimtaldehyd, 4-Hydroxy-3-methoxyzimtaldehyd (Coniferylaldehyd), 3,5-Dimethoxy-4-hydroxyzimtaldehyd sowie deren beliebigen Gemische eingesetzt werden.

4. Mittel nach einem der Ansprüche 1 bis 3, **dadurch gekennzeichnet, daß** als CH-aktive Verbindungen mit der Formel II 1,4-Dimethylchinolinium-, 1,2-Dimethylchinolinium-, 1,4-Dimethylpyridinium-, 1,2-Dimethylpyridinium-, 2-Methyl-1-ethyl-chinolinium-, 2,3-Di-methylisochinolinium-, 1,2,3,3-Tetramethyl-3H-indolium-, 2,3-Dimethylbenzothiazolium-, 2,3-Dimethyl-6-nitrobenzothiazolium-, 3-Benzyl-2-benzothiazolium-, 2-Methyl-3-propylbenzothiazolium-, 2,4-Dimethyl-3-ethylthiazolium-, 3-(2-Carboxyethyl)-2,5-dimethylbenzothiazolium-, 1,2,3-Trimethylbenzimidazolium-, 5,6-Dichlor-1,3-diethyl-2-methylbenzimidazolium-, 3-Ethyl-2-methylbenzothiazolium-, 3-Ethyl-2-methylnaphtho[1,2-d]thiazolium-, 5-Chlor-3-ethyl-2-methylbenzothiazolium-, 3-Ethyl-2-methylbenzoxazolium-Salze, die z. B. als Chloride, Bromide, lodide, Methansulfonate, Benzolsulfonate, p-Toluolsulfonate, Trifluormethansulfonate, Methylsulfate, Tetrafluorborate vorliegen können, sowie 2-Methyl-3-(3-sulfopropyl)-benzothiazolium-hydroxid, inneres Salz, 4-Methyl-1-(3-sulfopropyl)-pyridinium-hydroxid, inneres Salz, 4-Methyl-1-(3-sulfopropyl)-chinolinium-hydroxid, inneres Salz, 5-Methoxy-2-methyl-3-(3-sulfopropyl)-benzothiazolium-hydroxid, inneres Salz und beliebige Gemische der voranstehenden eingesetzt werden.

5. Mittel nach einem der Ansprüche 2 bis 4, **dadurch gekennzeichnet, daß** als CH-aktive Verbindungen mit der Formel III Rhodanin, Rhodanin-3-essigsäure, Barbitursäure, Thiobarbitursäure, 1,3-Dimethyl-, 1,3-Diethylthiobarbitursäure, Oxindol, 3-Indoxylacetat, Cumaranon, 1-Methyl-3-phenylpyrazolinon, Indan-1,3-dion, Cyclopentan-1,3-dion, 1,2-Dihydro-1-ethyl-6-hydroxy-4-methyl-2-oxo-3-pyridincarbonitril, 1-Dicyanmethylenindan, 1-Dicyanmethylenindan-3-on und beliebige Gemische der voranstehenden eingesetzt werden.

6. Mittel nach einem der Ansprüche 1 bis 5, **dadurch gekennzeichnet, daß** die aromatischen Aldehyde mit der Formel I und die quaternären heterocyclischen Verbindungen mit der Formel II und die CH-aktiven Verbindungen mit der Formel III jeweils in einer Menge von 0,03 bis 65 mmol, insbesondere von 1 bis 40 mmol, bezogen auf 100 g des gesamten Färbemittels, enthalten sind.

7. Mittel nach einem der Ansprüche 1 bis 6, **dadurch gekennzeichnet, daß** es Farbverstärker ausgewählt aus der Gruppe bestehend aus Piperidin, Piperidin-2-carbonsäure, Piperidin-3-carbonsäure, Piperidin-4-carbonsäure, Pyridin, 2-Hydroxypyridin, 3-Hydroxypyridin, 4-Hydroxypyridin, Imidazol, 1-Methylimidazol, Histidin, Pyrrolidin, Prolin, Pyrrolidon, Pyrrolidon-5-carbonsäure, Pyrazol, 1,2,4-Triazol, Piperazidin oder deren beliebigen Gemischen enthält.

8. Mittel nach einem der Ansprüche 1 bis 7, **dadurch gekennzeichnet, daß** es direkt ziehende Farbstoffe aus der Gruppe der Nitrophenylendiamine, Nitroaminophenole, Anthrachinone oder Indophenole vorzugsweise in einer Menge von 0,01 bis 20 Gew.-%, bezogen auf das gesamte Färbemittel, enthält.

9. Mittel nach einem der Ansprüche 1 bis 8, **dadurch gekennzeichnet, daß** Ammonium- oder Metallsalze ausgewählt aus der Gruppe der Formiate, Carbonate, Halogenide, Sulfate, Butyrate, Valeriate, Capronate, Acetate, Lactate, Glykolate, Tartrate, Citrate, Gluconate, Propionate, Phosphate und Phosphonate von Alkalimetallen, wie Kalium, Natrium oder Lithium, Erdalkalimetallen, wie Magnesium, Calcium, Strontium oder Barium, oder von Aluminium, Mangan, Eisen, Kobalt, Kupfer oder Zink, zugegeben werden.

10. Mittel nach einem der Ansprüche 1 bis 9, **dadurch gekennzeichnet, daß** es Oxidationsmittel, insbesondere H₂O₂, in einer Menge von 0,01 bis 6 Gew.-%, bezogen auf die Anwendungslösung, enthält.

11. Mittel nach einem der Ansprüche 1 bis 10, **dadurch gekennzeichnet, daß** es anionische, zwitterionische oder nichtionische Tenside enthält.

12. Verwendung von einer Kombination aus aromatischen Aldehyden mit der Formel I, 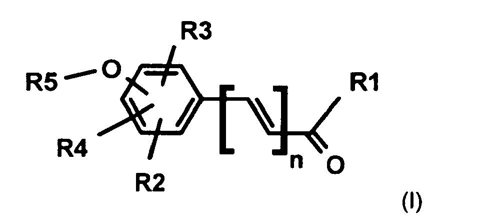 in der R¹ ein Wasserstoffatom, ist,
R², R³ und R⁴ unabhängig voneinander ein Wasserstoffatom, eine C₁₋₄-Alkylgruppe, ein Halogenatom, eine Hydroxy-, C₁₋₄-Alkoxy- oder Nitrogruppe bedeuten und zwei dieser Gruppen auch für einen ankondensierten aromatischen Ring stehen können,
R⁵ ein Wasserstoffatom, eine C₁₋₄-Alkyl-, C₂₋₄-Hydroxyalkyl-, C₁₋₄-Alkenyl- oder Arylgruppe bedeutet oder gemeinsam mit R², R³ oder R⁴ einen ankondensierten fünf- bis siebengliedrigen heterocylischen Ring bildet, oder gemeinsam mit dem Sauerstoffatom eine Olatgruppe bildet, deren negative Ladung durch ein Alkali- oder Ammoniumion ausgeglichen ist, und
n für die Zahlen 0, 1 oder 2 steht, und CH-aktiven Verbindungen mit der Formel II, 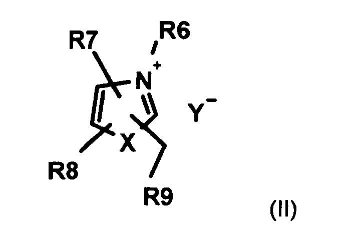 in der R⁶ eine C₁₋₁₀-Alkyl-, C₂₋₄-Alkenyl-, C₂₋₄-Hydroxyalkyl-, C₂₋₄-Carboxyalkyl-, C₂₋₄-Sulfoalkyl- oder Aralkylgruppe ist,
R⁷ und R⁸ unabhängig voneinander ein Wasserstoffatom, eine C₁₋₄-Alkylgruppe, ein Halogenatom, eine Hydroxy-, C₁₋₄-Alkoxy- oder Nitrogruppe bedeuten oder zusammen einen ankondensierten aromatischen Ring bilden,
R⁹ ein Wasserstoffatom, eine C₁₋₄-Alkyl- oder eine Arylgruppe,
X ein Sauerstoff- oder Schwefelatom, die Gruppe -CH=CH- oder >N-R¹², in der R¹² für eine C₁₋₄-Alkyl-, C₂₋₄-Carboxyalkyl-, C₂₋₄-Sulfoalkyl-, C₂₋₄-Sulfoxyalkyl-. C₂₋₄-Hydroxyalkyl- oder Aralkylgruppe steht, ist und
Y⁻ für ein Anion steht, das ausgewählt ist aus Halogenid, C₁₋₄-Alkylsulfat, C₁₋₄-Alkansulfonat, Arensulfonat, C₁₋₄-Perfluoralkansulfat, Tetrafluorborat, Perhalogenat, Sulfat, Hydrogensulfat oder Carboxylat, als eine färbende Komponente in Oxidationshaarfärbemitteln.

13. Verfahren zum Färben von keratinhaltigen Fasern, insbesondere menschlichen Haaren, worin ein Färbemittel, enthaltend mindestens eine Kombination aus aromatischen Aldehyden mit der Formel I, 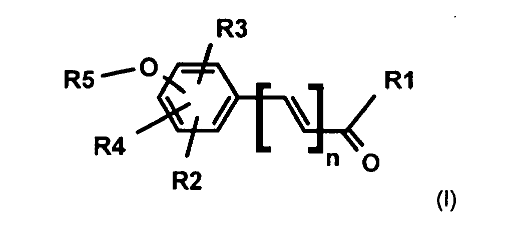 in der R¹ ein Wasserstoffatom, ist,
R², R³ und R⁴ unabhängig voneinander ein Wasserstoffatom, eine C₁₋₄-Alkylgruppe, ein Halogenatom, eine Hydroxy-, C₁₋₄-Alkoxy- oder Nitrogruppe bedeuten und zwei dieser Gruppen auch für einen ankondensierten aromatischen Ring stehen können,
R⁵ ein Wasserstoffatom, eine C₁₋₄-Alkyl-, C₂₋₄-Hydroxyalkyl-, C₁₋₄-Alkenyl- oder Arylgruppe bedeutet oder gemeinsam mit R², R³ oder R⁴ einen ankondensierten fünf- bis siebengliedrigen heterocylischen Ring bildet, oder gemeinsam mit dem Sauerstoffatom eine Olatgruppe bildet, deren negative Ladung durch ein Alkali- oder Ammoniumion ausgeglichen ist, und
n für die Zahlen 0, 1 oder 2 steht, und CH-aktiven Verbindungen mit der Formel II, 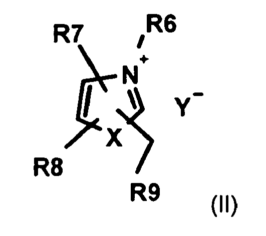 in der R⁶ eine C₁₋₁₀-Alkyl-, C₂₋₄-Alkenyl-, C₂₋₄-Hydroxyalkyl-, C₂₋₄-Carboxyalkyl-, C₂₋₄-Sulfoalkyl- oder Aralkylgruppe ist,
R⁷ und R⁸ unabhängig voneinander ein Wasserstoffatom, eine C₁₋₄-Alkylgruppe, ein Halogenatom, eine Hydroxy-, C₁₋₄-Alkoxy- oder Nitrogruppe bedeuten oder zusammen einen ankondensierten aromatischen Ring bilden,
R⁹ ein Wasserstoffatom, eine C₁₋₄-Alkyl- oder eine Arylgruppe,
X ein Sauerstoff- oder Schwefelatom, die Gruppe -CH=CH- oder >N-R¹², in der R¹² für eine C₁₋₄-Alkyl-, C₂₋₄-Carboxyalkyl-, C₂₋₄-Sulfoalkyl-, C₂₋₄-Sulfoxyalkyl-, C₂₋₄-Hydroxyalkyl- oder Aralkylgruppe stehen, ist und
Y- für ein Anion steht, das ausgewälhlt ist aus Halogenid, C₁₋₄-Alkylsulfat, C₁₋₄-Alkansulfonat, Arensulfonat, C₁₋₄-Perfluoralkansulfat, Tetrafluorborat, Perhalogenat, Sulfat, Hydrogensulfat oder Carboxylat, sowie übliche kosmetische Inhaltsstoffe, auf die keratinhaltigen Fasern aufgebracht, einige Zeit, üblicherweise ca. 30 Minuten, auf der Faser belassen und anschließend wieder ausgespült oder mit einem Shampoo ausgewaschen wird.

## Claims

1. Agent for dyeing keratin fibres, in particular human hair, comprising, as dyeing component, a combination of aromatic aldehydes with the formula I, 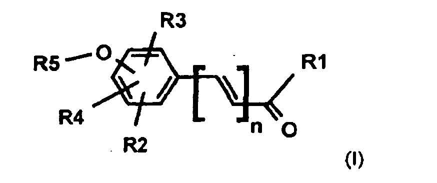 in which R¹ is a hydrogen atom,
R², R³ and R⁴, independently of one another, are a hydrogen atom, a C₁₋₄-alkyl group, a halogen atom, a hydroxy group, C₁₋₄-alkoxy group, or nitro group and two of these groups may also be a fused-on aromatic ring,
R⁵ is a hydrogen atom, a C₁₋₄-alkyl group, C₂₋₄-hydroxyalkyl group, C₁₋₄-alkenyl group or aryl group or, together with R², R³ or R⁴, forms a fused-on five- to seven-membered heterocyclic ring, or, together with the oxygen atom forms an olate group, whose negative charge is balanced by an alkali metal ion or ammonium ion, and
n is 0, 1 or 2 and CH-active compounds with the formula II, 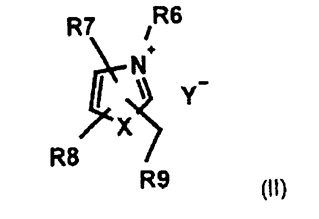 in which R⁶ is a C₁₋₁₀-alkyl group, C₂₋₄-alkenyl group, C₂₋₄-hydroxyalkyl group, C₂₋₄-carboxyalkyl group, C₂₋₄-sulphoalkyl group or aralkyl group,
R⁷ and R⁸, independently of one another, are a hydrogen atom, a C₁₋₄-alkyl group, a halogen atom, a hydroxy group, C₁₋₄-alkoxy group or nitro group or together form a fused-on aromatic ring,
R⁹ is a hydrogen atom, a C₁₋₄-alkyl group or an aryl group,
X is an oxygen or sulphur atom, the group, -CH=CH- or >N-R¹², in which R¹² is a C₁₋₄-alkyl, C₂₋₄-carboxyalkyl, C₂₋₄-sulphoalkyl, C₂₋₄-sulphoxyalkyl, C₂₋₄-hydroxyalkyl or aralkyl group, and
Y⁻ is an anion which is chosen from halide, C₁₋₄-alkyl sulphate, C₁₋₄-alkane-sulphonate, arenesulphonate, C₁₋₄-perfluoroalkane sulphate, tetrafluoroborate, perhalogenate, sulphate, hydrogen sulphate or carboxylate.

2. Agent according to Claim 1, **characterized in that** it additionally comprises CH-active compounds according to formula III, 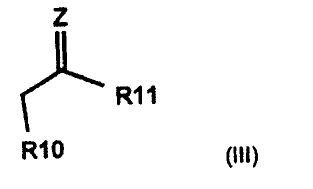 in which R¹⁰ is a C₁₋₄-acyl group, aroyl group, C₁₋₄-alkylsulphonyl group, C₁₋₄-alkylsulphinyl group, C₁₋₄-alkylamino group, di-C₁₋₄-alkylamino group, vinylcarbonyl group, methinimino group, nitrile group, ester group or carboxamide group, which may be optionally substituted by C₁₋₄-alkyl, C₂₋₄-hydroxyalkyl or aryl groups, and
R¹¹ is a C₁₋₄-acyl group, C₁₋₄-alkoxy group, C₁₋₄-alkylamino group, C₁₋₄-acylamino group or di-C₁₋₄-alkylamino group, where the radicals R¹⁰ and R¹¹, together with the remainder of the molecule, can form a 5-, 6- or 7-membered heterocycle from the series of thiazolidine-2,5-diones, thiazolidine-2-thion-5-ones, perhydropyrimidine-2,4,6-triones, perhydropyrimidine-2-thione-4,6-diones, 2-pyrazolin-5-ones, 1,2-dihydro-6-hydroxy-2-hydroxypyridines, benzothiazin-3-ones or enol esters thereof, and Z is oxygen, sulphur or the dicyanomethylene group

3. Agent according to Claim 1 or 2, **characterized in that** the compounds with the formula I used are salicylaldehyde, 3-hydroxybenzaldehyde, 4-hydroxybenzaldehyde, o-anisaldehyde, m-anisaldehyde, p-anisaldehyde, 4-hydroxy-3-methylbenzaldehyde, 2-hydroxy-3-methylbenzaldehyde, 2-hydroxy-5-methylbenzaldehyde, 2-hydroxy-4-methylbenzaldehyde, 2,3-dihydroxybenzaldenhyde, 2,5-dihydroxybenzaldehyde, 3,4-dihydroxybenzaldehyde, 2,4-dihydroxybenzaldehyde, 3,5-dihydroxybenzaldehyde, 2,3-dihydrobenzo[b]furan-5-carboxaldehyde, piperonal, 4-ethoxy-benzaldehyde, 3,5-dimethyl-4-hydroxybenzaldehyde, vanillin, isovanillin, 2,3,4-trihydroxybenzaldehyde, 2,4,5-trihydroxybenzaldehyde, 2,4,6-trihydroxybenzaldehyde, 3,4,5-trihydroxybenzaldehyde, 3-chloro-4-hydroxybenzaldehyde, 2,4-dimethoxybenzaldehyde, 2,5-dimethoxybenzaldehyde, 2,6-dimethoxybenzaldehyde, 3,4-dimethoxybenzaldehyde, 3,4-dihydroxy-5-methoxybenzaldehyde, 2-hydroxy-1-naphthaldehyde, 4-hydroxy-1-naphthaldehyde, 1-hydroxy-2-naphthaldehyde, 2-methoxy-1-naphthaldehyde, 4-methoxy-1-naphthaldehyde, 2-hydroxycinnamaldehyde, 4-hydroxycinnamaldehyde, 2,4-dihydroxycinnamaldehyde, 4-hydroxy-3-methoxycinnamaldehyde (coniferylaldehyde), 3,5-dimethoxy-4-hydroxyzimtaldehyde, and any mixtures thereof.

4. Agent according to one of Claims 1 to 3, **characterized in that** the CH-active compounds with the formula II used are the salts of 1,4-dimethylquinolinium, 1,2-dimethylquinolinium, 1,4-dimethylpyridinium, 1,2-dimethylpyridinium, 2-methyl-1-ethylquinolinium, 2,3-dimethylisoquinolinium, 1,2,3,3-tetramethyl-3H-indolium, 2,3-dimethylbenzothiazolium, 2,3-dimethyl-6-nitro-benzothiazolium, 3-benzyl-2-benzothiazolium, 2-methyl-3-propylbenzothiazolium, 2,4-dimethyl-3-ethylthiazolium, 3-(2-carboxyethyl)-2,5-dimethylbenzothiazolium, 1,2,3-trimethylbenzimidazolium, 5,6-dichloro-1,3-diethyl-2-methylbenzimidazolium, 3-ethyl-2-methylbenzothiazolium, 3-ethyl-2-methyl-naphtho[1,2-d]thiazolium, 5-chloro-3-ethyl-2-methylbenzothiazolium, 3-ethyl-2-methyl-benzoxazolium, which may be present, for example as chlorides, bromides, iodides, methane-sulphonates, benzenesulphonates, p-toluene-sulphonates, trifluoromethanesulphonates, methyl sulphates, tetrafluoroborates, and also 2-methyl-3-(3-sulphopropyl)benzothiazolium hydroxide, internal salt, 4-methyl-1-(3-sulphopropyl)pyridinium hydroxide, internal salt, 4-methyl-1-(3-sulphopropyl)quinolinium hydroxide, internal salt, 5-methoxy-2-methyl-3-(3-sulphopropyl)benzothiazolium hydroxide, internal salt and any mixtures of the above.

5. Agent according to one of Claims 2 to 4, **characterized in that** the CH-active compounds with the formula III used are rhodanine, rhodanine-3-acetic acid, barbituric acid, thiobarbituric acid, 1,3-dimethyl-, 1,3-diethylthiobarbituric acid, oxindole, 3-indoxyl acetate, cumaranone, 1-methyl-3-phenylpyrazolinone, indane-1,3-dione, cyclopentane-1,3-dione, 1,2-dihydro-1-ethyl-6-hydroxy-4-methyl-2-oxo-3-pyridinecarbonitrile, 1-dicyanomethyleneindane, 1-dicyanomethyleneindan-3-one and any mixtures of the above.

6. Agent according to one of Claims 1 to 5, **characterized in that** the aromatic aldehydes with the formula I and the quaternary heterocyclic compounds with the formula II and the CH-active compounds with the formula III are in each case present in an amount of from 0.03 to 65 mmol, in particular from 1 to 40 mmol, based on 100 g of the total dyeing agent.

7. Agent according to one of Claims 1 to 6, **characterized in that** it comprises colour enhancers chosen from the group consisting of piperidine, piperidine-2-carboxylic acid, piperidine-3-carboxylic acid, piperidine-4-carboxylic acid, pyridine, 2-hydroxypyridine, 3-hydroxypyridine, 4-hydroxypyridine, imidazole, 1-methylimidazole, histidine, pyrrolidine, proline, pyrrolidone, pyrrolidone-5-carboxylic acid, pyrazole, 1,2,4-triazole, piperazidine or any mixtures thereof.

8. Agent according to one of Claims 1 to 7, **characterized in that** it comprises direct dyes from the group of nitrophenylenediamines, nitroaminophenols, anthraquinones or indole-phenols, preferably in an amount of from 0.01 to 20% by weight, based on the total dyeing agent.

9. Agent according to one of Claims 1 to 8, **characterized in that** ammonium salts or metal salts chosen from the group of formates, carbonates, halides, sulphates, butyrates, valerates, caproates, acetates, lactates, glycolates, tartrates, citrates, gluconates, propionates, phosphates and phosphonates of alkali metals, such as potassium, sodium or lithium, alkaline earth metals, such as magnesium, calcium, strontium or barium, or of aluminium, manganese, iron, cobalt, copper or zinc, are added.

10. Agent according to one of Claims 1 to 9, **characterized in that** it comprises oxidizing agents, in particular H₂O₂, in an amount of from 0.01 to 6% by weight, based on the application solution.

11. Agent according to one of Claims 1 to 10, **characterized in that** it comprises anionic, zwitterionic or nonionic surfactants.

12. Use of a combination of aromatic aldehydes with the formula I, 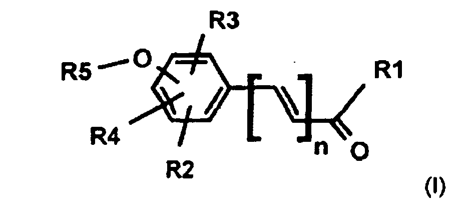 in which R¹ is a hydrogen atom,
R², and R³ R⁴, independently of one another, are a hydrogen atom, a C₁₋₄-alkyl group, a halogen atom, a hydroxy group, C₁₋₄-alkoxy group or nitro group and two of these groups may also be a fused-on aromatic ring,
R⁵ is a hydrogen atom, a C₁₋₄-alkyl group, C₂₋₄-hydroxyalkyl group, C₁₋₄-alkenyl group or aryl group or, together with R², R³, or R⁴, forms a fused-on five- to seven-membered heterocyclic ring, or, together with the oxygen atom forms an olate group, whose negative charge is balanced by an alkali metal ion or ammonium ion and
n is 0, 1 or 2 and CH-active compounds with the formula II, 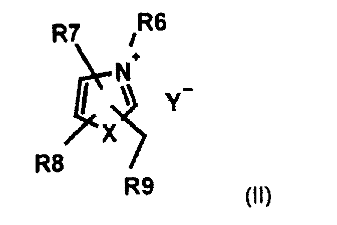 in which R⁶ is a C₁₋₁₀-alkyl group, C₂₋₄-alkenyl group, C₂₋₄-hydroxyalkyl group, C₂₋₄-carboxyalkyl group, C₂₋₄-sulphoalkyl group or aralkyl group,
R⁷ and R⁸, independently of one another, are a hydrogen atom, a C₁₋₄-alkyl group, a halogen atom, a hydroxy group, C₁₋₄-alkoxy group or nitro group or together form a fused-on aromatic ring,
R⁹ is a hydrogen atom, a C₁₋₄-alkyl group or an aryl group,
X is an oxygen or sulphur atom, the group, -CH=CH- or >N-R¹², in which R¹² is a C₁₋₄-alkyl, C₂₋₄-carboxyalkyl, C₂₋₄-sulphoalkyl, C₂₋₄-sulphoxyalkyl, C₂₋₄-hydroxyalkyl or aralkyl group, and
Y⁻ is an anion which is chosen from halide, C₁₋₄-alkyl sulphate, C₁₋₄-alkane-sulphonate, arenesulphonate, C₁₋₄-perfluoroalkane sulphate, tetrafluoroborate, perhalogenate, sulphate, hydrogen sulphate or carboxylate, as a dyeing component in oxidation hair colorants.

13. Method of dyeing keratin fibres, in particular human hair, in which a colorant, comprising at least one combination of aromatic aldehydes with the formula I, 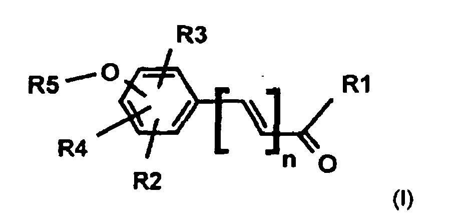 in which R¹ is a hydrogen atom,
R² R³ and R⁴, independently of one another, are a hydrogen atom, a C₁₋₄-alkyl group, a halogen atom, a hydroxy group, C₁₋₄-alkoxy group or nitro group and two of these groups may also be a fused-on aromatic ring,
R⁵ is a hydrogen atom, a C₁₋₄-alkyl group, C₂₋₄-hydroxyalkyl group, C₁₋₄-alkenyl group or aryl group or, together with R², R³ or R⁴, forms a fused-on five- to seven-membered heterocyclic ring, or, together with the oxygen atom forms an olate group, whose negative charge is balanced by an alkali metal ion or ammonium ion, n is 0, 1 or 2 and CH-active compounds with the formula II, 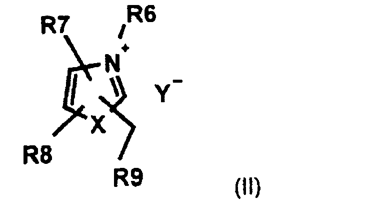 in which R⁶ is a C₁₋₁₀-alkyl group, C₂₋₄-alkenyl group, C₂₋₄-hydroxyalkyl group, C₂₋₄-carboxyalkyl group, C₂₋₄-sulphoalkyl group or aralkyl group,
R⁷ and R⁸, independently of one another, are a hydrogen atom, a C₁₋₄-alkyl group, a halogen atom, a hydroxy group, C₁₋₄-alkoxy group or nitro group or together form a fused-on aromatic ring,
R⁹ is a hydrogen atom, a C₁₋₄-alkyl group or an aryl group,
X is an oxygen or sulphur atom, the group -CH=CH-/>N-R¹², in which R¹² is a C₁₋₄-alkyl, C₂₋₄-carboxyalkyl, C₂₋₄-sulphoalkyl, C₂₋₄-sulphoxyalkyl, C₂₋₄-hydroxyalkyl or aralkyl group, and
Y⁻ is an anion which is chosen from halide, C₁₋₄-alkyl sulphate, C₁₋₄-alkane-sulphonate, arenesulphonate, C₁₋₄-perfluoroalkane sulphate, tetrafluoroborate, perhalogenate, sulphate, hydrogen sulphate or carboxylate, and customary cosmetic ingredients, is applied to the keratin fibres left for a time, usually about 30 minutes, on the fibres and then rinsed out again or washed out with a shampoo.

## Revendications

1. Agent pour teindre les fibres kératiniques, en particulier les cheveux humains, contenant en tant que composant colorant une combinaison d'aldéhydes aromatiques de formule I 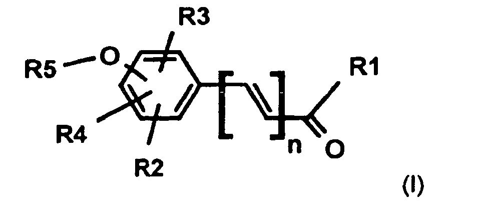 dans laquelle R¹ représente un atome d'hydrogène,
R², R³ et R⁴ représentent indépendamment les uns des autres, un atome d'hydrogène, un groupe alkyle en C₁ à C₄, un atome d'halogène ou un groupe hydroxy, alcoxy en C₁ à C₄, ou nitro et deux de ces groupes peuvent représenter également un noyau aromatique condensé,
R⁵ représente un atome d'hydrogène, un groupe alkyle en C₁ à C₄, un groupe hydroxyalkyle en C₂ à C₄, un groupe alcényle en C₁ à C₄ ou aryle
ou conjointement avec R², R³ ou R⁴ forme un cycle hétérocyclique de cinq à sept maillons condensé, ou conjointement avec l'atome d'oxygène forme un groupe oléate, dont la charge négative est compensée par un ion de métal alcalin ou ammonium, et n représente un nombre 0,1 ou 2,
et de composés à groupe CH actif de formule II, 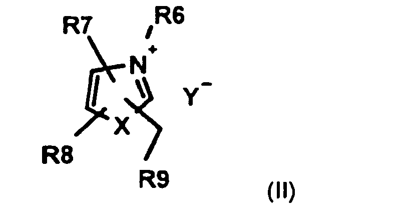 dans laquelle R⁶ représente un groupe alkyle en C₁ à C₁₀, alcényle en C₂ à C₄, hydroxyalkyle en C₂ à C₄, carboxyalkyle en C₂ à C₄, sulfoalkyle en C₂ à C₄ ou aralkyle,
R⁷ et R⁸ représentent indépendamment les uns des autres, un atome d'hydrogène, un groupe alkyle en C₁ à C₄, un atome d'halogène ou un groupe hydroxy, alcoxy en C₁ à C₄, ou nitro ou forment conjointement un noyau aromatique condensé,
R⁹ représente un atome d'hydrogène, un groupe alkyle en C₁ à C₄ ou aryle,
X représente un atome d'oxygène ou de soufre, le groupe -CH=CH- ou >N-R¹², dans lequel R¹² représente un groupe alkyle en C₁ à C₄, carboxyalkyle en C₂ à C₄, sulfoalkyle en C₂ à C₄, sulfoxyalkyle en C₂ à C₄, hydroxyalkyle en C₂ à C₄ ou aralkyle, et
Y⁻ représente un anion, qui est choisi parmi les ions halogénure, alkylsulfate en C₁ à C₄, alcane-sulfonate en C₁ à C₄, arène-sulfonate, perfluoro-alcane-sulfate en C₁ à C₄, tétrafluoroborate, perhalogénate, sulfate, hydrogéno-sulfate ou carboxylate.

2. Agent selon la revendication 1, **caractérisé en ce qu'**il contient en outre des composés à groupe CH actif de formule III, 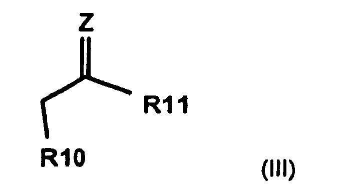 dans laquelle R¹⁰ représente un groupe acyle en C₁ à C₄, aroyle, alkylsulfonyle en C₁ à C₄, alkylsulfinyle en C₁ à C₄, alkylamino en C₁ à C₄, di-(alkylamino en C₁ à C₄), vinylcarbonyle, méthynimino, nitrile, ester ou amide d'acide carboxylique, qui peut être substitué éventuellement par des groupes alkyle en C₁ à C₄, hydroxyalkyle en C₂ à C₄ ou aryle, et
R¹¹ représente un groupe acyle en C₁ à C₄, alcoxy en C₁ à C₄, alkylamino en C₁ à C₄, acylamino en C₁ à C₄, ou di-(alkylamino en C₁ à C₄),
les radicaux R¹⁰ et R¹¹ pouvant former conjointement avec la molécule restante un hétérocycle de 5, 6 ou 7 maillons de la série des thiazolidine-2,5-diones, thiazolidine-2-thion-5-ones, perhydropyrimidine-2,4,6-triones, perhydropyrimidine-2-thione-4,6-diones, 2-pyrazolin-5-ones, 1,2-dihydro-6-hydroxy-2-hydroxypyridines, benzothiazin-3-ones ou leurs énolesters,
Z représente un atome d'oxygène, un atome de soufre ou le groupe dicyanométhylène.

3. Agent selon la revendication 1 ou 2, **caractérisé en ce que** le salicylaldéhyde, le 3-hydroxybenzaldéhyde, le 4-hydroxybenzaldéhyde, l'o-anisaldéhyde, le m-anisaldéhyde, le p-anisaldéhyde, le 4-hydroxy-3-méthylbenzaldéhyde, le 2-hydroxy-3-méthylbenzaldéhyde, le 2-hydroxy-5-méthylbenzaldéhyde, le 2-hydroxy-4-méthylbenzaldéhyde, le 2,3-dihydroxybenzaldéhyde, le 2,5-dihydroxybenzaldéhyde, le 3,4-dihydroxybenzaldéhyde, le 2,4-dihydroxybenzaldéhyde, le 3,5-dihydroxybenzaldéhyde, le 2,3-dihydrobenzo[b]furane-5-carboxaldéhyde, le pipéronal, le 4-éthoxy-benzaldéhyde, le 3,5-diméthyl-4-hydroxy-benzaldéhyde, la vanilline, l'isovanilline, le 2,3,4-trihydroxybenzaldéhyde, le 2,4,5-trihydroxybenzaldéhyde, le 2,4,6-trihydroxybenzaldéhyde, le 3,4,5-trihydroxybenzaldéhyde, le 3-chloro-4-hydroxy-benzaldéhyde, le 2,4-diméthoxybenzaldehyde, le 2,5-diméthoxybenzaldéhyde, le 2,6-diméthoxybenzaldéhyde, le 3,4-diméthoxybenzaldéhyde, le 3,4-dihydroxy-5-méthoxybenzaldéhyde, le 2-hydroxy-1-naphtaldéhyde, le 4-hydroxy-1-naphtaldéhyde, le 1-hydroxy-2-naphtaldéhyde, le 2-méthoxy-1-naphtaldéhyde, le 4-méthoxy-1-naphtaldéhyde, le 2-hydroxy-cinnamaldéhyde, le 4-hydroxycinnamaldéhyde, le 2,4-dihydroxycinnamaldéhyde, le 4-hydroxy-3-méthoxycinnamaldéhyde (coniférylaldéhyde), le 3,5-diméthoxy-4-hydroxycinnamaldéhyde ainsi que leurs mélanges, sont mis en oeuvre en tant que composés de formule I.

4. Agent selon l'une quelconque des revendications 1 à 3, **caractérisé en ce que** les sels de 1,4-diméthyl-quinoléinium, 1,2-diméthyl-quinoléinium, 1,4-diméthyl-pyridinium, 1,2-diméthyl-pyridinium, 2-méthyl-1-éthyl-quinoléinium, 2,3-diméthyl-isoquinoléinium, 1,2,3,3-tétraméthyl-3H-indolium, 2,3-diméthylbenzothiazolium, 2,3-diméthyl-6-nitro-benzothiazolium, 3-benzyl-2-benzothiazolium, 2-méthyl-3-propylbenzothiazolium, 2,4-diméthyl-3-éthylthiazolium, 3-(2-carboxyéthyl)-2,5-diméthylbenzothiazolium, 1,2,3-triméthylbenzimidazolium, 5,6-dichloro-1,3-diéthyl-2-méthyl-benzimidazolium, 3-éthyl-2-méthylbenzothiazolium, 3-éthyl-2-méthyl-naphto-[1,2-d]thiazolium, 5-chloro-3-éthyl-2-méthylbenzothiazolium, 3-éthyl-2-méthylbenzoxazolium, qui peuvent exister par exemple sous forme de chlorures, bromures, iodures, méthane-sulfonates, benzène-sulfonates, p-toluène-sulfonates, trifluorométhane-sulfonates, méthylsulfates, tétrafluoroborates, ainsi que l'hydroxyde de 2-méthyl-3-(3-sulfopropyl)-benzothiazolium, sel interne, l'hydroxyde de 4-méthyl-1-(3-sulfopropyl)-pyridinium, sel interne, l'hydroxyde de 4-méthyl-1-(3-sulfopropyl)-quinoléinium, sel interne, l'hydroxyde de 5-méthoxy-2-méthyl-3-(3-sulfopropyl)-benzothiazolium, sel interne, et des mélanges quelconques des précédents sont mis en oeuvre en tant que composés à groupe CH actif de formule II.

5. Agent selon l'une quelconque des revendications 2 à 4, **caractérisé en ce que** la rhodanine, l'acide rhodanine-3-acétique, l'acide barbiturique, l'acide thiobarbiturique, l'acide 1,3-diméthylthiobarbiturique, l'acide 1,3-diéthylthiobarbiturique, l'oxindol, l'acétate de 3-indoxyle, la coumaranone, la 1-méthyl-3-phényl-pyrazolinone, l'indane-1,3-dione, la cyclopentane-1,3-dione, le 1,2-dihydro-1-éthyl-6-hydroxy-4-méthyl-2-oxo-3-pyridine-carbonitrile, le 1-dicyanométhylène-indane, la 1-dicyanométhylène-indan-3-one, et des mélanges quelconques des précédents sont mis en oeuvre en tant que composés à groupe CH actif de formule III.

6. Agent selon l'une quelconque des revendications 1 à 5, **caractérisé en ce que** les aldéhydes aromatiques de formule I et les composés hétérocycliques quaternaires de formule II et les composés à groupe CH actif de formule III sont contenus à chaque fois en une quantité de 0,03 à 65 mmoles, en particulier de 1 à 40 mmoles, par rapport à 100 g de la teinture totale.

7. Agent selon l'une quelconque des revendications 1 à 6, **caractérisé en ce qu'**il contient des renforçateurs de couleur choisis dans le groupe formé par la pipéridine, l'acide pipéridine-2-carboxylique, l'acide pipéridine-3-carboxylique, l'acide pipéridine-4-carboxylique, la pyridine, la 2-hydroxypyridine, la 3-hydroxypyridine, la 4-hydroxypyridine, l'imidazole, le 1-méthylimidazole, l'histidine, la pyrrolidine, la proline, la pyrrolidone, l'acide pyrrolidone-5-carboxylique, le pyrazole, le 1,2,4-triazole, la pipérazidine ou leurs mélanges quelconques.

8. Agent selon l'une quelconque des revendications 1 à 7, **caractérisé en ce qu'**il contient des colorants montant directement sur les fibres du groupe des nitrophénylènediamines, des nitroaminophénols, des anthraquinones ou des indophénols, de préférence en une quantité de 0,01 à 20% en poids, par rapport à la teinture totale

9. Agent selon l'une quelconque des revendications 1 à 8, **caractérisé en ce que** des sels d'ammonium ou métalliques choisis dans le groupe formé par les formiates, les carbonates, les halogénures, les sulfates, les butyrates, les valériates, les caproates, les acétates, les lactates, les glycolates, les tartrates, les citrates, les gluconates, les propionates, les phosphates et les phosphonates de métaux alcalins, comme le potassium, le sodium ou le lithium, de métaux alcalino-terreux comme le magnésium, le calcium, le strontium ou le baryum ou d'aluminium, de manganèse, de fer, de cobalt, de cuivre ou de zinc, sont ajoutés.

10. Agent selon l'une quelconque des revendications 1 à 9, **caractérisé en ce qu'**il contient un agent d'oxydation, en particulier H₂O₂, en une quantité de 0,01 à 6% en poids, par rapport à la solution d'application.

11. Agent selon l'une quelconque des revendications 1 à 10, **caractérisé en ce qu'**il contient des tensio-actifs anioniques, zwitterioniques ou non ioniques.

12. Utilisation d'une combinaison d'aldéhydes aromatiques de formule I, 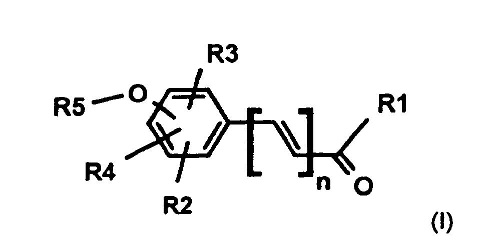 dans laquelle R¹ représente un atome d'hydrogène,
R², R³ et R⁴ représentent indépendamment les uns des autres, un atome d'hydrogène, un groupe alkyle en C₁ à C₄, un atome d'halogène ou un groupe hydroxy, alcoxy en C₁ à C₄, ou nitro et deux de ces groupes peuvent représenter également un noyau aromatique condensé,
R⁵ représente un atome d'hydrogène, un groupe alkyle en C₁ à C₄, un groupe hydroxyalkyle en C₂ à C₄, un groupe alcényle en C₁ à C₄ ou aryle
ou conjointement avec R², R³ ou R⁴ forme un cycle hétérocyclique de cinq à sept maillons condensé, ou conjointement avec l'atome d'oxygène forme un groupe oléate, dont la charge négative est compensée par un ion de métal alcalin ou ammonium, et n représente un nombre 0,1 ou 2,
et de composés à groupe CH actif de formule II, 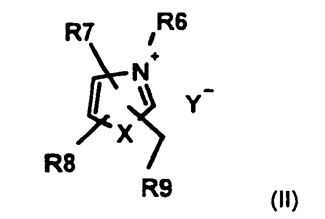 dans laquelle R⁶ représente un groupe alkyle en C₁ à C₁₀, alcényle en C₂ à C₄, hydroxyalkyle en C₂ à C₄, carboxyalkyle en C₂ à C₄, sulfoalkyle en C₂ à C₄ ou aralkyle,
R⁷ et R⁸ représentent indépendamment les uns des autres, un atome d'hydrogène, un groupe alkyle en C₁ à C₄, un atome d'halogène ou un groupe hydroxy, alcoxy en C₁ à C₄, ou nitro, ou forment conjointement un noyau aromatique condensé,
R⁹ représente un atome d'hydrogène, un groupe alkyle en C₁ à C₄ ou aryle,
X représente un atome d'oxygène ou de soufre, le groupe -CH=CH- ou >N-R¹², dans lequel R¹² représente un groupe alkyle en C₁ à C₄, carboxyalkyle en C₂ à C₄, sulfoalkyle en C₂ à C₄, sulfoxyalkyle en C₂ à C₄, hydroxyalkyle en C₂ à C₄ ou aralkyle, et
Y⁻ représente un anion, qui est choisi parmi les ions halogénure, alkylsulfate en C₁ à C₄, alcane-sulfonate en C₁ à C₄, arène-sulfonate, perfluoro-alcane-sulfate en C₁ à C₄, tétrafluoroborate, perhalogénate, sulfate, hydrogénosulfate ou carboxylate,
en tant que composant colorant dans des teintures capillaires par oxydation.

13. Procédé pour teindre les fibres kératiniques, en particulier les cheveux humains, dans lequel une teinture contenant au moins une combinaison d'aldéhydes aromatiques de formule I, 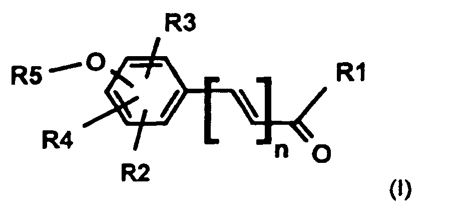 dans laquelle R¹ représente un atome d'hydrogène,
R², R³ et R⁴ représentent indépendamment les uns des autres, un atome d'hydrogène, un groupe alkyle en C₁ à C₄, un atome d'halogène ou un groupe hydroxy, alcoxy en C₁ à C₄, ou nitro et deux de ces groupes peuvent représenter également un noyau aromatique condensé,
R⁵ représente un atome d'hydrogène, un groupe alkyle en C₁ à C₄, un groupe hydroxyalkyle en C₂ à C₄, un groupe alcényle en C₂ à C₄ ou aryle
ou conjointement avec R², R³ ou R⁴ forme un cycle hétérocyclique de cinq à sept maillons condensé, ou conjointement avec l'atome d'oxygène forme un groupe oléate, dont la charge négative est compensée par un ion de métal alcalin ou ammonium, et n représente un nombre 0,1 ou 2,
et de composés à groupe CH actif de formule II, 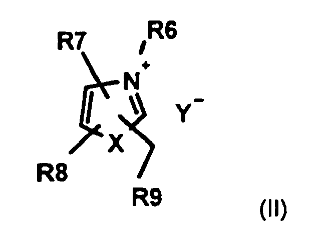 dans laquelle R⁶ représente un groupe alkyle en C₁ à C₁₀, alcényle en C₂ à C₄, hydroxyalkyle en C₂ à C₄, carboxyalkyle en C₂ à C₄, sulfoalkyle en C₂ à C₄ ou aralkyle,
R⁷ et R⁸ représentent indépendamment les uns des autres, un atome d'hydrogène, un groupe alkyle en C₁ à C₄, un atome d'halogène ou un groupe hydroxy, alcoxy en C₁ à C₄, ou nitro, ou forment conjointement un noyau aromatique condensé,
R⁹ représente un atome d'hydrogène, un groupe alkyle en C₁ à C₄ ou aryle,
X représente un atome d'oxygène ou de soufre, le groupe -CH=CH- ou >N-R¹², dans lequel R¹² représente un groupe alkyle en C₁ à C₄, carboxyalkyle en C₂ à C₄, sulfoalkyle en C₂ à C₄, sulfoxyalkyle en C₂ à C₄, hydroxyalkyle en C₂ à C₄ ou aralkyle, et
Y⁻ représente un anion, qui est choisi parmi les ions halogénure, alkylsulfate en C₁ à C₄, alcane-sulfonate en C₁ à C₄, arène-sulfonate, perfluoro-alcane-sulfate en C₁ à C₄, tétrafluoroborate, perhalogénate, sulfate, hydrogénosulfate ou carboxylate,
ainsi que des ingrédients cosmétiques habituels, est appliquée sur les fibres kératiniques, et laissée quelque temps, habituellement environ 30 minutes sur la fibre, puis éliminée par rinçage ou par lavage avec un shampoing.
